# EUROPEAN PATENT APPLICATION

(11) **EP 4 155 394 A1**
(43) Date of publication of application: **29.03.2023**
(21) Application number: 21807602.4
(22) Date of filing: 17.05.2021
(51) Int. Cl.: C12N 9/02, C12N 15/09

(54) **USE OF LYTIC POLYSACCHARIDE MONOOXYGENASES, ENZYMATIC COMPOSITION CONTAINING SAME, AND DEGRADATION METHOD FOR PLASTIC POLYMERS**

(30) Priority: 18.05.2020 BR 102020009936; 14.05.2021 BR 102021009404
(71) Applicant: CNPEM (CENTRO NACIONAL DE PESQUISA EM ENERGIA E MATERIAIS, 13.083-970 Campinas (BR)
(72) Inventor: MURAKAMI, Mário Tyago, 13083100 Campinas (BR); RIBEIRO CORRÊA, Thamy Lívia, 13083-100 Campinas (BR)
(74) Representative: Petraz, Gilberto Luigi
(86) International application number: PCT/BR2021/050203
(87) International publication number: WO 2021/232124

(57) **Abstract**

The present invention relates to the novel activity of the enzymatic composition containing lytic polysaccharide monooxygenases (LPMOs) that are bacterial (Auxiliary Activity 10, AA10) and/or fungal (Auxiliary Activity 9, AA9) for degrading polyethylene terephthalate (PET) and related plastic polymers. The genes that encode *Kp*LPMO10A (AA10) and *Af*LPMO9A (AA9) were isolated from *Kitasatospora papulosa* and *Aspergillus fischeri* microorganisms, respectively. Methods such as atomic force microscopy (AFM) and X-ray photoelectron spectroscopy (XPS) detected alterations in the superficial chemical composition and morphology of the PET found in liquid bottles when treated with LPMOs. The gentle temperature conditions used during the LPMO-PET reaction suit the use of these enzymes to help canonical enzymes (PETases) deconstruct plastics, which is beneficial for the circular economy for PET.

## Description

### SEQUENCE LISTING REFERENCE

This application contains a sequence listing in computer readable form. The computer readable form is incorporated herein by reference.

### FIELD OF THE INVENTION

The present invention belongs to the broad chemical and environmental areas, specifically in the areas of biodegradation, bioremediation and biofragmentation.

### BACKGROUND OF THE INVENTION

Plastic is found in several areas of industry, with diverse applications, such as food packaging, pharmaceuticals, toys, general household products, construction, decoration and countless other industries. Each plastic material has a different chemical composition, some of which are reusable and others have a complex recycling process.

Plastic polymers are classified into two large distinct groups according to processing and thermal behavior: thermoplastics and thermosets. The former are moldable, as they soften when heated; and the second are not easily moldable by heating (Spinac6 and de Paoli, A tecnologia da reciclagem de polimeros. Quimica Nova, 2005, 28 (1), 65-72).

Among the plastics that are produced and used for various commercial purposes, there are polyethylene terephthalate (PET or PETE or polyester), high density polyethylene (HDPE or HDPE), polyvinyl chloride (PVC), low density polyethylene (LDPE or LDPE), polypropylene (PP), polystyrene (PS, styrofoam), polyurethane (PU), polytetrafluoroethylene (Teflon) and other plastics that can be laminated or mixed with other types of polyesters.

A survey carried out in 2018 showed that the main plastic materials consumed in Brazil are PP (20.3%), HDPE (13.5%) and LDPE (11.4%). PET represents 5.9% of national consumption. In 2012, the PET recycling rate in Brazil was 58% (Abiplast 2019, available at http://www.abiplast.org.br/wp-content/uploads/2019/10/perfil2018-web_VC.pdf; https ://sidra.ibge.gov.br/tabela/1202#resultado, visited on April 2, 2020).

PET is a semi-aromatic thermoplastic. It is a semi-crystalline, hygroscopic and colorless polymer with excellent coating ability, good tensile strength and impact resistance. It is one of the most abundant and manufactured polyester plastics worldwide, having numerous applications: prefabricated containers, thermoformed products, textile sector, furniture, medical implants and automotive, aviation and aerospace industries.

One of the main applications of PET is in the field of packaging such as bottles or films, due to its excellent water, gas and moisture damming properties, in addition to its low permeability (Koshti, Mehta and Samarth, Biological Recycling of Polyethylene Terephthalate: A mini-review, Journal of Polymers and the Environment 2018, 26, 3520-3529).

Since the plastic industry emerged, the production of this product has reached a total of 8.3 billion tons in the world and, of this amount produced, most of it is used only once and immediately discarded, in general, in sanitary landfills.

In 2018, the United Nations (UN) started a global awareness movement about the disposal of plastic objects in the environment and its effects. One of the main aggravating factors of pollution caused by plastic materials is due to the high half-life of this material, which sometimes takes more than 200 years to decompose in nature, which causes a strong environmental impact, especially when discarded in the oceans. In the marine environment, plastic affects the beings of this ecosystem by altering biochemical cycles inherent to marine flora due to blocking the penetration of oxygen and light into the sea. In addition, turtles and fish can feed on plastic material, causing them to die. Around 91% of the plastic used worldwide is not recycled.

According to data from the World Bank (2018), Brazil is the fourth largest producer of plastic waste in the world, with 11.3 million tons, behind only the United States (70 million tons), China (54.7 million tons), tonnes) and India (19.3 million tonnes). More than 10.3 million tons of this total waste generated are collected (91 %), but only 145 thousand tons (1.28 %) are effectively recycled, considered one of the lowest rates in the survey and well below the global average of plastic recycling, which is 9%. Generally, waste is disposed of irregularly, where cooperatives work with little efficiency, since about 10% of the waste they receive is non-reusable waste (Kaza et al. Word Bank Group 2018, What a waste 2.0 - A Global Snapshot of Solid Waste Management to 2050, v.1, 275p).

Even in recycling plants, there are losses during the categorization of types of plastics because they are contaminated, multilayered or of low value. The destination of 7.7 million tons of plastic is the sanitary landfill; while another 2.4 million tons are discarded irregularly, without any type of treatment, in an open pit.

Plastic recycling can be classified as primary, secondary, tertiary and quaternary. The primary is the reintroduction of scrap and polymer fragments into the cycle to produce similar products with characteristics similar to the original products (Pinto, A. G. Lixo Municipal: Manual de Gerenciamento Integrado, IPT/CEMPRE 2018, 4a ed, 351p). The secondary one deals with the mechanical reprocessing of simple polymeric materials and involves the following steps: crushing, separation, washing and extrusion (Al-Salem, Lettieri e Baeyens, The valorization of plastic solid waste (PSW) by primary to quaternary routes: From re-use to energy and chemicals, Progress in Energy and Combustion Science 2010, 36, 103-129). Tertiary, or chemistry, involves the process of depolymerization by thermochemical processes (pyrolysis, catalytic conversion) (Pinto, A. G. Lixo Municipal: Manual de Gerenciamento Integrado, IPT/CEMPRE 2018, 4aed, 351p; Al-Salem, Lettieri e Baeyens, The valorization of plastic solid waste (PSW) by primary to quaternary routes: From re-use to energy and chemicals, Progress in Energy and Combustion Science 2010, 36, 103-129; Hopewell, Dvorak e Kosior, Plastics recycling: challenges and opportunities. Philosofical Transactions of the Royal Society B 2009, 364, 2115-2126). Chemical recycling is an alternative to plastic waste that has exhausted its recycling capacity by the secondary method and to plastic, laminated and multilayer films. (British Plastics Federation, Plastics Recycling. <http://www.bpf.co.uk/sustainability/plastics_recycling.aspx>, visited on April 2, 2020). A quaternária, ou recuperação energética de residues, acontece pelo método de incineração gerando vapor ou energia, reduzindo o volume do material em 90 a 99 % em volume e diminuindo o descarte em aterro. Entretanto, esse método deve ser monitorado devido ao decorrente impacto ambiental (Al-Salem, Lettieri e Baeyens, The valorization of plastic solid waste (PSW) by primary to quaternary routes: From re-use to energy and chemicals, Progress in Energy and Combustion Science, 2010, 36, 103-129; Hopewell, Dvorak and Kosior, Plastics recycling: challenges and opportunities. Philosofical Transactions of the Royal Society B 2009, 364, 2115-2126).

Another well-known route of plastic degradation, known as weathering or photodegradation, involves exposure to UV light, in addition to mechanical changes caused by waves and winds or crushing in rocks and marine sediments, which eventually break larger plastics into smaller pieces known as micro- (> 5 mm) and nanoplastics (0.1 µm). However, these smaller particles are likely to be incorporated into the food chain and end up in the intestines of animals, including man, although this has not yet been determined by case study (Danso, Chow and Streit, Plastics: Environmental and biotechnological perspectives on microbial degradation. Applied and Environmental Microbiology 2019, 85 (19), 1095-1109).

The PET, which is a functional unit between terephthalic acid (AT) and ethylene glycol (EG), is, as a rule, recycled in the following ways: 1) the material is granulated, cleaned and extruded to become a PET bottle again ; or 2) PET is broken down into its monomers which can be re-polymerized. Both approaches are conducted at high temperatures.

Thus, generally, the main recycling methods require high temperature and high pressure conditions (chemical methods) or consume a large amount of energy and/or generate a large amount of toxic and harmful substances to the environment, which also results in secondary pollution (biological).

To minimize these effects, based on an environmentally sustainable approach to the recycling of plastic products, biodegradation methods have been intensively applied and studied. (Ma et al, Enhanced Poly(ethylene terephthalate) hydrolase activity engineering. Engineering 2018, 4, 888-891; Yoshida et al, A bacterium that degrades and assimilates poly(ethylene terephthalate. Science 2016, 351 (6278), 1196-1199).

Recent research on biodegradation has addressed the ability of microorganisms to degrade plastic. With the current knowledge on plastic degradation through microbial means, it is known that enzymes secreted or obtained from microorganisms act mainly on polyethylene terephthalate (PET) and polyurethane (PU) polymers. However, the best enzymes and microorganisms with activity in PU and PET still show moderate catalysis rates.

Generally, microbial degradation of polymers is a slow process. This high resistance derives mainly from the fiber's high molecular weight, strong C-C interactions and the extremely hydrophobic surface, which is very difficult for enzymes to attack. Furthermore, polymers have different molecular orderings (e.g. amorphous and crystalline forms), resulting in different levels of degradability. (Danso, Chow e Streit, Plastics: Environmental and biotechnologycal pespectives on microbial degradation. Applied and Environmental Microbiology 2019, 85 (19), 1095-1109).

The first evidence of biodegradation of aromatic polyesters was reported in a study published in 2005, in which the microorganism *Thermobifida fusca* was used to degrade PET (Müller et al, Enzymatic degradation of Poly(ethylene terephthalate): rapid hydrolyse using a hydrolase form T. fusca. Macromolecular Rapid Communication 2005, 26, 1400-1405). From then on, other microorganisms were tested in the degradation of this substrate.

For example, patent document JP2000143868 describes the method of degrading aromatic polyester with microorganisms *Trichosporon* FEM BP-6445 and *Arthrobacter* FERM BP-6444.

Patent document WO2005019439 describes the genus *Rhizobium,* in particular *Rhizobium sp.* OKH-03, having the ability to degrade aromatic polyesters.

Patent document JP20081999957 describes gram negative bacillus 201-F6 isolated from soil with aromatic polyester degradation activity.

One approach to biodegradation consists of the use of enzymes isolated from microorganisms. Currently, there are 27 known functional and verified enzymes, most of which are biochemically broken down according to the synthetic polymer substrates or oligomers/monomers to which they bind. Cutinases enzymes represent the largest dominant fraction within the studies performed.

For example, enzymes have been reported *Thermobifida alba* Cut1 (ADV92525), *Thermobifida fusca* Cut2 (CBY05530), *Thermobifida fusca* Cut1 (AET05798), *Thermobifida halotolerans* Serine hydrolase (AFA45122), *Saccharomonospora viridis* Cutinase (BAO42836), *Thermomonospora curvata* triacilglicerol lipase(WP 012851645), LCC (AEV21261), *Ideonella sakaiensis* PETase (GAP38373), *Polyangium brachysporum* triacilglicerol lipase (WP047194864), *Vibrio gazogenes* lipase (WP021018894), LiplAF5-2 (ACC95208), *Oleispira antarctica* LipA (CCk74972), *Ideonella sakaiensis* MHETase (WP 082368715), *Humicola insolens* cutinase (4OYY) and *Fusarium oxysporum cutinase* (5AJH) degrade polyethylene substrates. For degradation of the polyamide (PA) substrate, enzymes have been applied *Arthrobacter* sp NylE (WP079941038), *Arthrobacter* sp NylA (BAA05090), *Arthrobacter* sp NylB (CAA24927) and *Arthrobacter* sp NylC (YP001965068). The enzymes *Pseudomonas oleovorans* AHs (CAB54050), *Pseudomonas fluorescens* StyB (CAB06823), *Pseudomonas fluorescens* StyA (CAB06823) and *Pseudomonas fluorescens* StyC (CAB06825) degraded polystyrene (PS), while the degradation of polyurethane (PU) can occur with *Pseudomonas fluorescens* PueA (AAC23718), *Pseudomonas fluorescens* PueB (AAY92474) and *Pseudomonas fluorescens* PulA (AAF66684) (Danso, Chow and Streit, Plastics: Environmental and biotechnological perspectives on microbial degradation. Applied Environmental Microbiology 2019, 85 (19), 1095-1109).

The enzymatic deconstruction of polyethylene terephthalate (PET) has been the subject of extensive previous research and it can be grouped into two categories: (1) the enzymatic modification of the surface of the polyester or (2) the depolymerization into their respective monomers. Different enzymes with specific properties are needed for these two processes. Enzymatic surface modification is possible with several families of enzymes, such as lipases, carboxylesterases, cutinases and proteases. On the other hand, obtaining monomers from PET requires substantial degradation of the PET components; therefore, only a limited number of cutinases, recognized as PETases, act by releasing monomers from PET. The first PET hydrolase was discovered by Müller et al. (Enzymatic degradation of poly(ethylene terephthalate): rapid hydrolyse using hydrolase from T. fusca. Macromolecular Rapid Communications 2005, 26, 1400-1405).

Therefore, there is a demand for enzymes that present PET degradation activity and that can be used industrially in the recycling of this material.

The lytic polysaccharide monooxygenases, or LPMOs, constitute a recently identified group of enzymes. These enzymes were originally classified as a GH (glycosyl hydrolase) or CBM (carbohydrate binding module) family, and now belong to the auxiliary activity enzymes (AAs), identified under the classes AA9 - AA11 and AA13 - AA16 in the CAZy databases. It is known that the presence of LPMOs in an enzyme cocktail can increase the activity of canonical GHs by up to two orders of magnitude. In addition to cellulose, LPMOs oxidize a wide range of saccharide-based polymers, including hemicellulose, starch, and chitin. Notably, some LPMOs exhibit substrate promiscuity. In general, LPMOs increase the performance of GHs by acting in regions where GHs do not act, increasing the repertoire of ends to be recognized by canonical GHs (Chen et al. Enzymatic degradation of plant biomass and synthetic polymers. Nature Reviews Chemistry 2020, 4: 114-126).

*Kp*LPMO10A is an LPMO obtained from *Kitasatospora papulosa* and cloned into *Escherichia coli,* and its use to degrade lignocellulosic biomass has been described. (Corrêa et al, An actinobacteria lytic polysaccharide Monooxigenase acts on both cellulose and xylan to boost biomass saccharification. Biotechnology for Biofuels 2019, 12:117).

Although the catalytic mechanism and applications of LPMOs have attracted the industrial interest in recent years, the use of this class of enzymes as biodegradation agents for plastics has barely been explored.

Only the document WO201412506 was identified, addressing the use of glycosyl hydrolase 61 (GH61), the former denomination of AA9, originating from several genera of bacteria (gram positive:*Bacillus* sp. and *Streptomyces* sp. or gram negative: *E.coli* or *Pseudomonas* sp.) and fungi (*Candida, Kluyveromyces, Pichia, Saccharomyces, Schizosaccharomyces* or *Yarrowia* or filamentous fungi) associated with cutinases for the treatment of fabrics based on polyester (PET). The inventors have demonstrated that the combination of enzymes shows greater tissue degradation than with cutinases alone. However, the document does not address the isolated use of LPMOs in the degradation of PET, much less in the degradation of plastic waste from bottles or jars. Furthermore, the described enzymatic degradation process occurred at high temperatures (between 65 and 90 °C), as commonly reported for PET recycling.

Thus, there is a demand to provide an alternative to the enzymatic degradation of polyethylene terephthalate (PET) that is advantageous over the alternatives described in the prior art.

Furthermore, it was not found prior art documents that refer to or suggests an enzymatic degradation process by lytic polysaccharide monooxygenases (LPMOs) isolated from the bacterium *Kitasatospora papulosa* and the filamentous fungus *Aspergillus fischeri,* whose the only known function until now is to hydrolyze lignocellulosic compounds, which can be used in the degradation of polyester plastic materials without the aid of canonical enzymes.

### BRIEF DESCRIPTION OF THE INVENTION

The present invention describes a new use of enzymes belonging to the family of *Lytic Polysaccharide Mono-oxygenases -* LPMOs. More precisely, the present invention describes the use of LPMOs in the degradation of polyethylene, more specifically, in the degradation of polyethylene terephthalate (PET).

The LPMOs of the present invention are isolated from microorganisms, preferably being isolated from bacteria of the genus *Kitasatospora* sp. and from fungi of the genus *Aspergillus* sp.

In one embodiment, the present invention provides an enzyme composition for use in degrading polyester which comprises at least one lytic polysaccharide monooxygenase of the present invention and an acceptable vehicle. In a specific embodiment, the composition of the present invention additionally comprises a cutinase, more specifically a PETase.

In a second embodiment, the present invention provides the use of the enzyme composition of the present invention in the degradation of polyethylene terephthalate (PET).

In a third embodiment, the present invention provides a method of degrading PET which comprises the use of at least one lytic polysaccharide monooxygenase of the present invention. In a specific embodiment, the method of the present invention further comprises employing a cutinase, more specifically a PETase.

Finally, in a fourth embodiment, the present invention provides the use of at least one lytic polysaccharide monooxygenase in the enzymatic degradation of polyester, wherein said lytic polysaccharide monooxygenase degrades said polyester without the aid of at least one canonical enzyme. In a preferred embodiment, the lytic polysaccharide monooxygenases of the present invention are used.

### BRIEF DESCRIPTION OF THE DRAWINGS

Figure 1 shows the expression vector for *Kp*LMO10A (or LPMO AA10) from the *Kitasatospora papulosa* (A) and *Af*LPMO9A (or LPMO AA9) from the *Aspergillus fischeri* (B).
Figure 2 shows the protein profile of *Kp*LPMO10A (A), *Af*LPMO9A (B) and IsPETase (C) obtained from two chromatographic steps.
Figure 3 shows images obtained by Atomic Force Microscopy of PET Mylar films treated with LPMOs. A: untreated control; B: film treated with *Kp*LPMO10A; D: film treated with A*f*LPMO10A.
Figure 4 shows the images obtained by Atomic Force Microscopy of PET bottles treated with LPMOs. A, untreated control; B, PET treated with *Kp*LPMO10A; C, PET treated with A*f*LPMO9A.
Figure 5 shows the spectra obtained by the XPS technique of the control (A), PET bottle post enzymatic degradation by A*f*LPMO9A.
Figure 6 shows the prediction of possible products generated by LPMOs from PET.

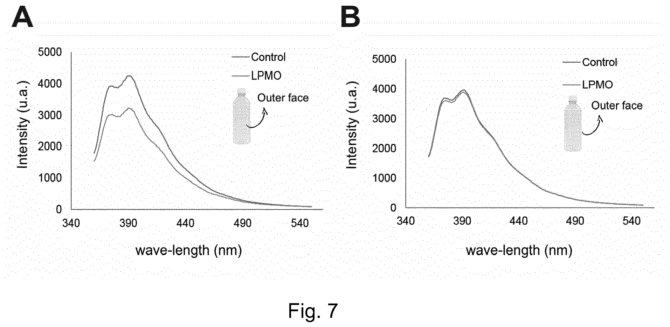

Figure 7 shows the fluorescence emission spectra of PET bottle cards treated and untreated with the enzymes of the present invention. (A): inner face. (B): external face. The material was excited at 340 nm.

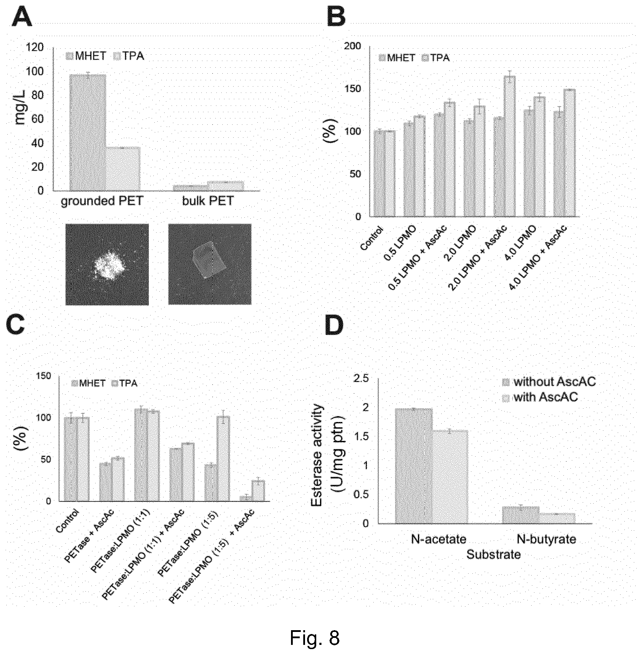

Figure 8 shows the results of synergism tests between *Af*LPMO9A (LPMO) and *Is*PETase (PETase) on PET, with measurements of mono (2-hydroxyethyl) terephthalate (MHET) and terephthalic acid (TPA) release. (A) Activity of *Is*PETase on a block of grounded PET from bottles. (B) MHET and TPA release by *Is*PETase from pre-treated grounded PET with different concentrations of A*f*LPMO9A. (C) MHET and TPA release by *Is*PETase in reactions with simultaneous addition of *Is*PETase and *Af*LPMO9A in different concentrations. (D) *Is*PETase activity on N-acetate and N-butyrate in the absence and presence of ascorbic acid.

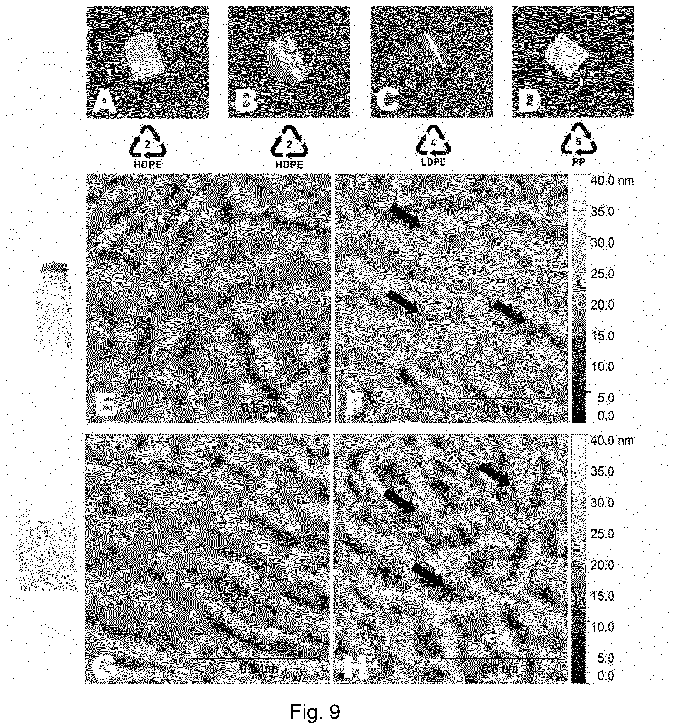

Figure 9 shows topographic images of atomic force microscopy of a juice bottle and a plastic bag, both made of HDPE, treated with AscAC (E and G, control) and in the presence of A*f*LPMO9A (F and H). The arrows point to erosions on the material, caused by enzymatic action.

### DETAILED DESCRIPTION OF THE INVENTION

The present invention describes a new use of enzymes belonging to the family of Lytic Polysaccharide Monooxygenases - LPMOs.

When used in its isolated form, in other words, without the presence of enzymes with other functions, the enzymes of the LPMOs family are known only for their activity in the oxidation of polysaccharides, such as cellulose, hemicellulose, starch and chitin.

The inventors of the present invention found that LPMOs have polyethylene degradation activity, which is surprising in light of the activities of these enzymes described so far, demonstrating their use in the degradation of plastics, more precisely in the degradation of polyethylene terephthalate (PET).

Equally surprising, the inventors of the present invention have demonstrated that the LPMOs of the present invention exhibit PET-degrading activity when isolated, i.e, without the need for the concomitant use of canonical enzymes such as cutinases or PETases.

Furthermore, the inventors of the present invention have demonstrated that the association of the LPMOs of the present invention with canonical enzymes has a surprising synergistic effect on PET degradation.

The inventors of the present invention also developed a polyethylene degradation method employing the LPMOs of the present invention, which occurs at 30-37 °C, being advantageous in relation to the other methods of enzymatic degradation of polyethylene described in the state of the art.

The LPMOs of the present invention comprise an amino acid sequence that shows at least 60% identity with the sequences SEQ ID NO: 2 or SEQ ID NO: 5.

The LPMOs of the present invention, comprising at least 60% identity to the sequences SEQ ID NO: 2 or SEQ ID NO: 5, can be isolated from bacteria or fungi. The bacterial LPMOs include *Bacillus* sp., *Burkholderia* sp., *Caldibacillus* sp., *Cellvibrio* sp., *Enterococcus* sp., *Hahella* sp., *Jonesia* sp., *Listeria* sp., *Micromonospora* sp., *Nocardiopsis* sp., *Photorhabdus* sp., *Serratia* sp., *Teredinibacter* sp., *Thermobifida* sp. and *Vibrio* sp.; more specifically, *Streptomyces* sp., e.g., *Streptomyces ambofaciens, Streptomyces coelicolor, Streptomyces griseus, Streptomyces lividans, Streptomyces pratensis, Streptomyces atroolivaceus, Streptomyces swartbergensis, Streptomyces azureus, Streptomyces afghaniensis, Streptomyces coeruleoribdus, Streptomyces pristinaespiralis, Streptomyces coelicoflavus, Streptomyces pactum, Streptomyces qaidamensis, Streptomyces olivaceus, Streptomyces africanus, Streptomyces iakyrus, Streptomyces regalis, Streptomyces luteus, Streptomyces cyaneogriseus, Streptomyces havaiiensis, Streptomyces rubrogriseus, Streptomyces marokkonensis, Streptomyces corynorhini, Streptomyces pharetrae, Streptomyces glauscescens* e *Streptomyces capillispiralis;* more specifically, K*itasatospora* sp., e.g., *Kitasatospora cheerisanensis, Kitasatospora aureofaciens, Kitasatospora aburaviensis, Kitasatospora herbaricolor, Kitasatospora misakiensis, Kitasatospora xanthocidica* and *Kitasatospora papulosa.* Fungal LPMOs include those from *Gloeophyllum* sp., *Heterobasidium* sp., *Pleurotus* sp., *Pestalotiopsis* sp., *Phanerochaete* sp., *Podospora* sp., *Lentinus* sp., *Miceliophythora* sp., *Neurospora* sp., *Trametes* sp., *Thermoascus* sp., *Thermothelomyces* sp. and *Trichoderma* sp.; more specifically, *Penicillium,* e.g., *Penicillium oxalicum, Penicillium subrubescens, Penicillium rolfsii, Penicillium brasilianum, Penicillium antarcticum, Penicillium arizonense, Penicillium steckii, Penicillium vulpinum, Penicillium expansum, Penicillium camemberti, Penicillium solitum, Penicillium coprophilum, Penicillium roqueforti, Penicillium polonicum, Penicillium freii, Penicillium rubens, Penicillium griseofulvum, Penicillium digitatum, Penicillium italicum* e *Penicillium nalgiovense;* mais especificamente, *Aspergillus* sp., por exemplo, *Aspergillus lentulus, Aspergillus fumigatus, Aspergillus novofumigatus, Aspergillus turcosus, Aspergillus thermomutatus, Aspergillus udagawae, Aspergillus clavatus, Aspergillus homomorphus, Aspergillus aculeatinus, Aspergillus fijiensis, Aspergillus brunneoviolaceus, Aspergillus uvacrum, Aspergillus aculeatus, Aspergillus tanneri, Aspergillus brasiliensis, Aspergillus terreus, Aspergillus tamarii, Aspergillus nidulans, Aspergillus caelatus, Aspergillus ibericus, Aspergillus piperis, Aspergillus japonicus, Aspergillus niger, Aspergillus oryzae, Aspergillus flavus, Aspergillus kawachii, Aspergillus saccharolyticus, Aspergillus neoniger, Aspergillus glaucus, Aspergillus ruber, Aspergillus tubingensis, Aspergillus leporis, Aspergillus mulundensis* and *Aspergillus fischeri.*

The LPMOs of the present invention are preferably derived from bacteria of the genus *Kitasatospora* sp. and fungi of the genus *Aspergillus* sp. Preferably, the enzymes of the present invention are derived from the bacterium *Kitasatospora papulosa,* such as the enzyme *Kp*LPMO10A, and the filamentous fungus *Aspergillus fischeri,* such as the enzyme *Af*LPMO9A.

The *Kp*LPMO10A polypeptide chain consists of amino acids 39 to 224 of SEQ ID NO: 2 spanning the N-terminal histidine (H39) typical of LPMOs and H146 and Y215 related to the coordination of the metal ion, which may be a copper.

The *Af*LPMO9A polypeptide chain consists of amino acids 20-247 of SEQ ID NO: 5 spanning the N-terminal histidine (H20) typical of LPMOs and H105 and Y194 possibly related to the coordination of the metal ion, which may be a copper.

The enzymes of the present invention can be obtained by transforming expression vectors into cells using steps known in the art. For example, the enzymes of the present invention are expressed in cells of *Escherichia coli* containing suitable expression vectors grown in media and under suitable conditions and extracted by lysis using a suitable protocol. Then, the enzymes of the present invention are obtained using separation techniques such as, for example, affinity chromatography, with the protein profile of the fractions obtained being evaluated on an SDS-PAGE gel. Additional steps for purifying the enzymes of the present invention can be applied employing suitable techniques, such as gel filtration chromatography using a suitable column.

In one embodiment, the present invention provides an enzyme composition for use in degrading polyester that comprises at least one lytic polysaccharide monooxygenase of the present invention (SEQ ID NO: 2 or SEQ ID NO: 5) (LPMO), and an acceptable carrier.

An acceptable vehicle, within the scope of the present invention, is understood as an aqueous vehicle that maintains the enzymes of the present invention in suspension or solution when in adequate concentration, and may contain, in addition to water, other additives, such as preservatives, agents or buffering systems, and so forth.

The LPMOs of the present invention are present in a concentration of between approximately 0.001% and approximately 0.1% by total weight of the composition of the present invention, preferably between approximately 0.002% and approximately 0.005% by total weight of the composition.

The compositions of the present invention contain at least one LPMO of the present invention, which may contain an isolated enzyme or a combination of enzymes within the context of the invention.

In a specific embodiment, the composition of the present invention additionally comprises a cutinase, more specifically a PETase.

Cutinases are enzymes related to the degradation of aliphatic polyester cutin, found in the plant cuticle. All PET hydrolases (PETases) that were characterized so far belong to the cutinases family. PETases have a wider catalytic center when compared to cutinases, probably related to the accommodation of semi-aromatic crystalline polyesters. Polyesterases act preferentially in amorphous regions of PET, unlike LPMOs which act preferentially in crystalline regions on polymeric substrates where their activity has been previously reported. Therefore, the joint action of LPMO-PETase can be advantageous as reported for LPMOs-GHs.

Polyesters and polyamides recognized by cutinases include poly(L-lactic acid) (PLA), polyethylene furanoate (PEF), polybutylene adipate-co-terephthalate (PBAT), polycaprolactone (PCL), polybutylene succinate (PBS) , polyamide 6,6 and polyethylene terephthalate (PET).

Cutinases with PET hydrolase (PETase) activity to be used in the composition of the present invention can be of fungal or bacterial origin. Those of fungal origin include those originating from *Humicola* sp., *Fusarium* sp., *Aspergillus* sp. and *Penicillium* sp. Bacterial include those from *Acidovorax* sp., *Rhizobacter* sp., *Pseudomonas* sp., *Streptomyces* sp., *Thermobifida* sp., *Saccharomonospora* sp., *Clostridium* and *Ideonela* sp., specifically, *Ideonela sakaiensis.*

The concentration of PETase in the composition of the present invention ranges from approximately 0.0005% to approximately 0.05% by total weight of the composition, preferably from approximately 0.001 to approximately 0.0025% by total weight of the composition.

In a second embodiment, the present invention provides the use of the enzyme composition of the present invention in the degradation of plastic polymers, more specifically, polyethylene.

According to the present invention, plastic waste used as a substrate for enzymatic degradation can be selected fromPEAD, PVC, PEBD, PP, PS, PC, PU, ABS, PE and PET, preferably PU, ABS, PE e PET, more preferably ABS, PE e PET, even more preferably PE and PET, and even more preferably PET.

Among the waste that can be called PET, there are liquid bottles, food packaging, window films, X-ray films, fabric fibers and any other materials mostly made of PET, without limitations.

In a third embodiment, the present invention provides a method of degrading PET which comprises the use of at least one lytic polysaccharide monooxygenase of the present invention.

Surprisingly, the inventors of the present invention have identified that LPMOs can be employed alone for the deconstruction of PET.

The PET degradation method of the present invention comprises adding at least one LPMO of the present invention in an aqueous medium containing substrate.

The concentration of LPMO to be used depends on the origin and conditions of pH and temperature of the reaction, being initially standardized according to methods that are familiar to the person skilled in the art.

The concentration of LPMO in the reactions of the present invention is between 0.01-50 milligrams of LPMO per gram of PET, preferably 1-30 milligrams of LPMO per gram of PET, preferably 2-20 milligrams of LPMO per gram of PET.

The substrate concentration in the form of PET in the process of the present invention varies between 10-80% of the reaction, preferably 20-70% of the reaction, preferably 40-60% of the reaction, preferably 30-50% of the reaction.

In a preferred embodiment, the aqueous medium additionally comprises a buffer solution.

The concentration of the buffer solution is between 0.01-0.5M, preferably 0.03-0.25M, preferably 0.05-0.1M.

In a preferred embodiment, the PET degradation method of the present invention comprises an additional step, wherein an electron donating substance for LPMO is added.

According to the present invention, the electron donating molecule for LPMO can be selected from ascorbic acid and mixtures thereof.

The concentration of the electron donor molecule for LPMO varies between 0.3mM-2mM, preferably 0.8-1.5mM, preferably 0.5-1mM.

The reaction time of the present invention is usually between 5 minutes and 48 hours, preferably between 10 minutes and 48 hours, more preferably between 30 minutes and 48 hours, and even more preferably between 60 minutes and 48 hours.

The pH of the reaction is selected according to the origin of the LPMO used and is previously standardized, it can be between pH 4.0-9.0, preferably between pH 5.0-8.0 and, more preferably, between pH 5.5-7.5.

The reaction temperature is selected according to the origin of the LPMO used and is previously standardized. The reaction is conducted at temperatures between 20 and 60 °C, preferably 25 and 55 °C, preferably between 30 and 50 °C In a specific embodiment, the temperature employed in the method of the present invention is below 40 °C, preferably 37 °C.

In a specific embodiment, the method of the present invention additionally comprises employing a cutinase.

The PETase concentration to be used depends on the reaction conditions, being initially standardized. The concentration of PETase in the reactions of the present invention can be between 0.005 and 25 milligrams of PETase per gram of PET, preferably between 0.5 and 15 milligrams of PETase per gram of PET, preferably between 1 and 10 milligrams of PETase per gram of PET.

In the context of the invention, it is a composition of enzymes comprising a lytic polysaccharide monooxygenase (LPMO), with recognized ability to oxidize the crystalline portion of the glucan structure favoring the action of (hemi)cellulases on lignocellulose substrates, and /or blending with the hydrolase (PETase) for the potential degradation of plastic polymers in the depolymerisation/recycling industry.

The following examples illustrate preferred embodiments of the present invention. They do not, therefore, limit the scope of protection of the invention, which is exclusively defined by the claims accompanying this description.

### Example 1: Preparation of expression vector construct for lytic polysaccharide monooxygenases of the present invention

STEP 1: The bacteria *K. papulosa* (DSM4643) had the genomic DNA extracted and used for amplification of the gene of interest *Kp*LPMO10A (SEQ ID NO: 1, nucleotide 115-672), which encodes the AA10 enzyme *Kp*LPMO10A, with primers KpF and KpR (Table 1). The amplification product was used for a new PCR reaction with the KpFpET22b and KpRpET22b primers (Table 1) for the addition of homology tails to the pET22b vector. The gene was cloned into the pET22b(+) vector immediately after the sequence encoding the *pel*B signal peptide.

**Table 1. Primers used for the amplification of gene KpLPMO10A**

| Name | Sequence (5'-3') |
|---|---|
| KpF | CACGGTTCCGTCGTCGAC |
| KpR | GGTGAAGTTCACGTCACTGCAC |
| KpFpET22b | CTGCCCAGCCGGCGATGGCCCACGGTTCCGTCGTCGAC |
| KpRpET22b | TCAGTGGTGGTGGTGGTGGTGGGTGAAGTTCACGTCACT GCAC |

STEP 2: The gene *AfLPMO9A* (SEQ ID NO: 3, nucleotide 58-741), which encodes the AA9 enzyme *Af*LPMO9A, was added with sequence encoding pelB (SEQ ID NO: 4, nucleotide 1-66) and had the codons optimized for expression in *Escherichia coli.* The *pel*B + *AfLPMO9A* was synthesized and cloned into the restriction sites for *Ndel* and *Xhol* enzymes in the pET21a(+) vector by Genscript (Piscataway).

STEP 3: The *Is*PETase gene (GenBank: BBYR01000074.1), which encodes the PETase enzyme in *Ideonella sakaiensis,* had the codons optimized for expression in *E*. *coli.* The *Is*PETase was synthesized and cloned into the restriction sites for *Ndel* and *Xho*l enzymes in the pET21b(+) vector by Genscript (Piscataway).

STEP 4: The vectors pET22b(+)-*KpLPMO10A* and pET21a(+)-*AfLPMO9A* were transformed (separately) into cells of *Escherichia coli* Shuffle^{®}. The cells were cultivated in TB medium and added with IPTG to promote protein expression. *Kp*LPMO10A (SEQ ID NO: 2, amino acid 39-224) or *Af*LPMO9A (SEQ ID NO: 2, amino acid 20-247) for 16 h at 18°C/250 rpm. Cells were centrifuged and lysed by osmotic shock protocol. The supernatants obtained from the lysis were loaded onto a 5mL His-Trap affinity column (GE) for chromatography. The protein profile of fractions obtained by affinity chromatography was evaluated on SDS-PAGE gel. Fractions containing proteins of the expected size were mixed, concentrated and used for gel filtration chromatography using HiLoad Superdex G-75 16/60 column. Fractions containing pure proteins of the expected size (assessed by SDS-PAGE) were pooled, concentrated and assessed for concentration (mg protein/mL). *Kp*LPMO10A or *Af*LPMO9A were treated with copper sulfate (CuSO₄) for 16 hours at 4 °C. Excess metal was removed using a Sephadex G-25 PD-10 column. *Kp*LPMO10A or *Af*LPMO9A they were concentrated, quantified (mg of protein/mL) and used in later experiments.

### Example 2: Preparation of expression vector construct for PET hydrolase of the present invention

STEP 1: The pET21b(+)-*Is*PETase vector was transformed into *Escherichia coli* BL21(DE3) cells. The cells were cultivated in LB medium and added with IPTG to promote the expression of *Is*PETase durante 16 h a 18 °C/250 rpm. Cells were centrifuged and lysed with the aid of lysozyme and sonicator. The supernatants obtained from the lysis were loaded onto a 5mL His-Trap affinity column (GE) for chromatography. The protein profile of fractions obtained by affinity chromatography was evaluated on SDS-PAGE gel. Fractions containing proteins of the expected size were pooled, concentrated and used for gel filtration chromatography using HiLoad Superdex G-75 16/60 column. Fractions containing pure proteins of the expected size (assessed by SDS-PAGE) were pooled, concentrated and evaluated for concentration (mg protein/mL) and used in further experiments.

### Example 3: Preparation of polyester film for enzymatic reactions

STAGE 1: PET, Mylar and bottle PET were cut into dimensions of 0.6 cm × 0.4 cm using scissors. Subsequently, they were washed with 1 mL of Milli-Q water using a pipette and subjected to ultrasound for 5 minutes. This procedure was performed 3 times. The plastics were dried in a N₂ stream for 10-15 minutes and stored for later use.

### Example 4: Enzymatic degradation of waste polyester films

STEP 1: Assays that aid the direct detection of products released by LPMOs from plastic polymers are not available. The reaction conditions initially adopted are the same used for (hemi)cellulose substrates. The enzymatic reactions were carried out in 2 mL Eppendorf tubes containing PET (Mylar or bottle PET), 0.05 M sodium phosphate buffer, pH 6.0, 20 mg of *Kp*LPMO10A or *Af*LPMO9A per gram of PET. After 10 minutes at 37 °C, the reactions were spiked with 1 mM ascorbic acid and incubated at 37 °C, 850 rpm for 48 hours.

The PET from the enzymatic treatment was rinsed with 0.5 mL of Milli-Q water using a pipette and subjected to ultrasound for 5 minutes. This procedure was performed 3 times. The liquid fraction of the reactions was boiled at 99 °C for 10 minutes and the material was saved for later analysis.

### Example 5: Characterization measurements of the polyester under study

### a. Atomic Force Microscopy

The PET from the enzymatic treatment was submitted to analysis by atomic force microscopy using the Multimode8 model and the NanoScope V controller (Bruker, Germany) operating in PeakForce tapping mode with probes model ScanAsyst-air (Bruker, Germany) of silicon nitride with a nominal force of 0.4 Nm⁻¹. The images obtained (3x3 µm) were treated in the Gwyddion software. b. *X-ray photoelectron spectroscopy* analysis (XPS)

XPS spectra were obtained with a VSW HA100 spectrometer (United Kingdom) operated in constant pass energy mode, set at 44eV. As excitation, radiation from an aluminum anode was used, which produces photons with 1486.6 eV of energy. High-resolution spectra were collected with a step close to 0.1 eV and sufficient accumulation time to have a good signal-to-noise ratio. The pressure in the analysis chamber was kept below 6×10⁻⁸ mBar during the analysis.

The samples were fixed to a stainless steel sample holder with double-sided carbon tape and introduced into the analysis chamber.

The electrical charge of the samples was corrected using the value of the benzene ring at 284.7 eV. The deconvolution was carried out using Gaussians. The images were obtained in the software Origin.

The genes that code for proteins *Kp*LPMO10A (AA10) (SEQ ID NO: 1) and *Af*LPMO9A (AA9) (SEQ ID NO: 2) were isolated from the bacteria *Kitasatospora papulosa* and from the filamentous fungi *Aspergillus fischeri,* respectively.

*Kitasatospora* and *Streptomyces* have similar morphologies, but differ in cell wall composition, characterizing *Kitasatospora* as a distinct group within the *Streptomyces. Kitasatospora* proteins can be characterized as belonging to *Streptomyces sp.* due to the high identity of amino acid sequences between the two genera.

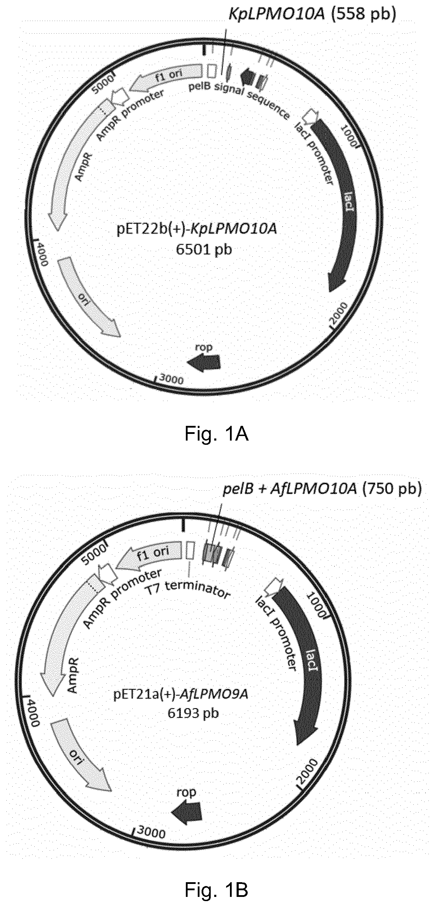

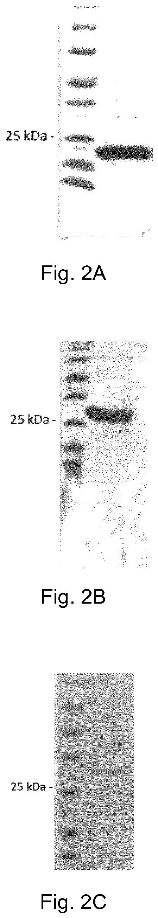

*Kp*LPMO10A, *Af*LPMO9A (from SEQ ID NO: 1 to SEQ ID NO: 5) and *Is*PETase were expressed by *E. coli* cells transformed with the vectors pET22b(+)-*Kp*LPMO10A, pET21a(+)-*Af*LPMO9A (Figure 1) and pET21a(+)-*Is*PETase, respectively and purified by two chromatographic steps (Figure 2).

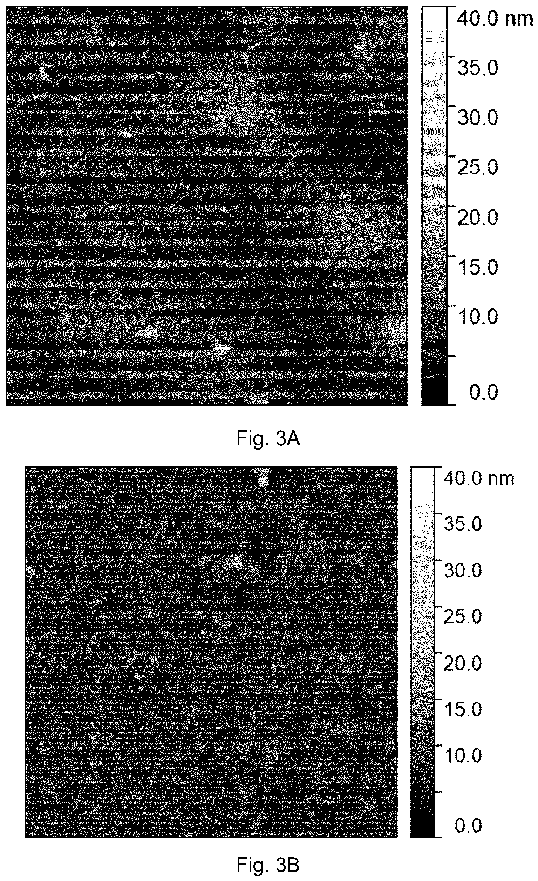

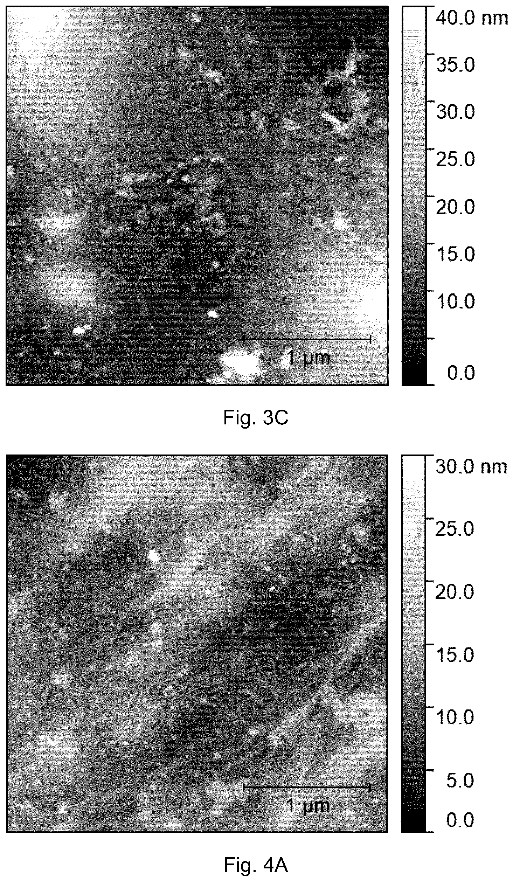

The activity of *Kp*LPMO10A and *Af*LPMO9A on PET film (Mylar) was evaluated by atomic force microscopy. Using the atomic force microscopy technique, the topography of a sample is obtained after scanning its surface with a probe, if any surface alteration is detected. Reactions conducted with *Kp*LPMO10A (Figure 3B) and *Af*LPMO9A (Figure 3C) led to changes (erosions) on the surface of the PET film when compared to the control reaction conducted in the absence of the enzyme (Figure 3A). The effect was more intense when *Af*LPMO9A was employed in the reaction (Figure 3C).

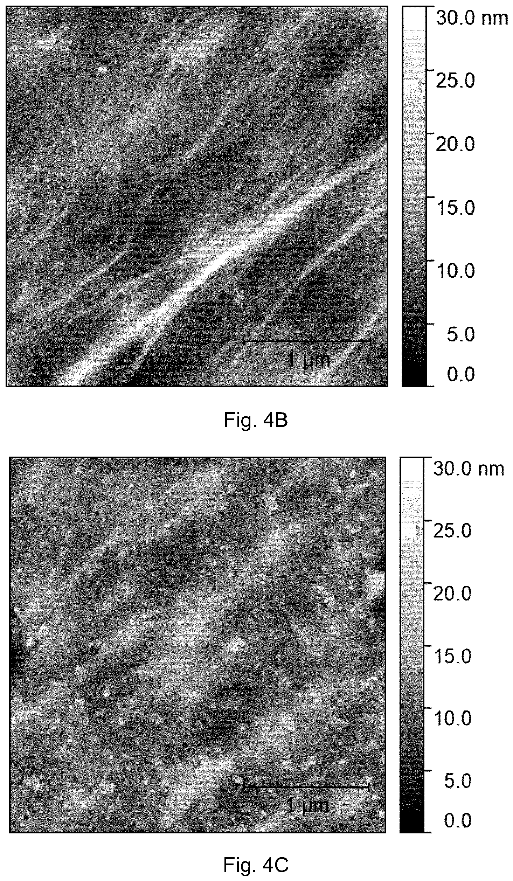

The activity of *Kp*LPMO10A and *Af*LPMO9A was evaluated using PET from bottles as substrate, in the same way as it was for the PET film (Mylar). Compared to the control (Figure 4A), reaction conducted in the absence of enzymes, *Kp*LPMO10A (Figure 4B) and *Af*LPMO9A (Figure 4C) altered the surface of PET bottles, and the effect (of erosion or "peeling" of PET) of *Af*LPMO9A was more intense when compared to *Kp*LPMO10A. The same enzyme concentration was adopted for *Kp*LPMO10A and *Af*LPMO9A in both experiments.

In view of the changes noted on the surface of PET mainly in reactions containing *Af*LPMO9A, the enzyme was selected for further experiments. Residual bottle PET obtained after treatment with *Af*LPMO9A was subjected to *X-ray photoelectron spectroscopy* analysis (XPS). The spectra obtained by XPS are a powerful tool to evaluate chemical alterations contained in the surface of materials, in this case, PET.

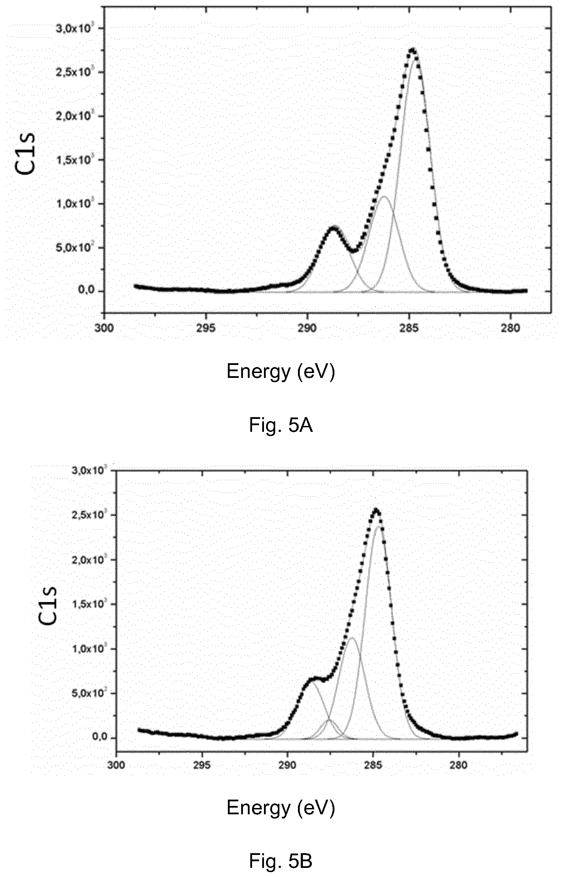

The intensity of C1-related peaks for various chemical states found on the PET surface are shown in Figure 5A (Control, PET not treated with *Af*LPMO9A) and Figure 5B (PET treated with *Af*LPMO9A) and Table 2. The deconvolution of the control sample spectra and treated with *Af*LPMO9A resulted in three peaks, mainly in the regions (eV): 284.7, bonds contained in benzene rings (aromatic carbons); 286.2, methylene carbon and 288.6, carboxyl ester, consistent with other studies carried out on the surface of PET. The treatment of the sample with *Af*LPMO9A led to a drop in the intensity of the peak corresponding to 284.7 eV and the appearance of the peak at 287.5 eV, which corresponds to the formation of double bonds between carbon and oxygen atoms (C=O) on the PET surface (Table 2).

**Table 2. Intensity of peaks related to C1 for different chemical states on the PET surface**

| Treatment | Bonds | | | |
|---|---|---|---|---|
| Energy | COO (288,6) | C=O (287,5) | C-O/CN (286,2) | CH_{X},C-C,C=C (284,7) |
| Control | 16,81 | X | 24,2 | 58,9 |
| *Af*LPMO9A | 14,96 | 3,27 | 26,3 | 55,4 |

The LPMOs introduce an oxygen atom at carbon 1 (C1) or carbon 4 (C4) of the glucose molecule when cellulose is used as substrate resulting in the release of aldonic acid and ketoaldose, respectively. Furthermore, some LPMOs have been shown to be capable of releasing a mixture of both C1 and C4 oxidized products from the same substrate.

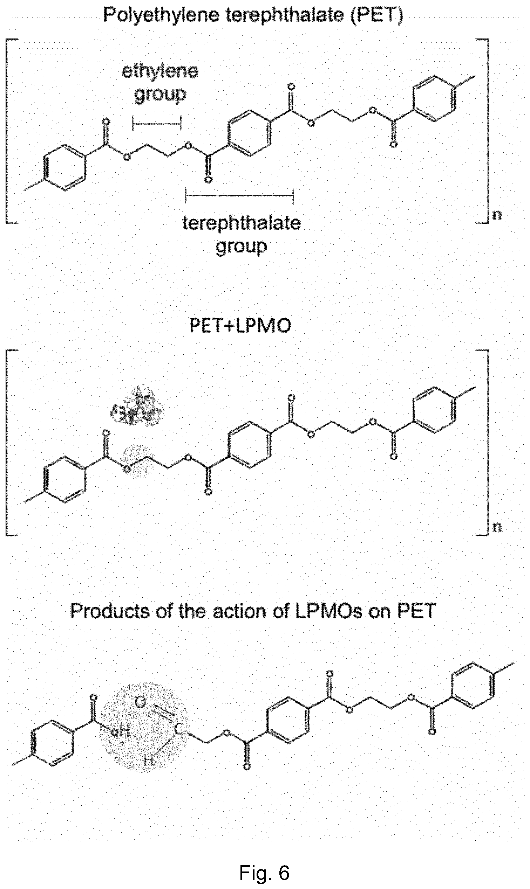

Based on the products released by LPMOs from cellulose, Figure 6 highlights the possible products released from PET polymer by LPMOs.

### Example 6: Fluorescence Assay

Fluorescence emission spectra of PET bottle cards treated and not treated with the enzymes of the present invention were obtained at room temperature in a Hitachi F-4500 FL spectrophotometer, with excitation at 340 nm and collecting the emission between 360 and 550 nm. The peaks at 370 nm (excimer) and 390 nm (ground state dimer) recorded in cards treated with LPMO excited at 340 nm showed reduced emission in relation to the control treated with AscAc, indicating the occurrence of scission of the PET chain. No decrease in peak emissions of 370/390 was observed when exciting the outer face (exposed to air) of the LPMO-treated PET bottle, which agrees with the atomic force microscopy data discussed above.

### Example 7: PETase Synergy Assay

The synergism between *Af*LPMO9A and *Is*PETase was evaluated by non-simultaneous or simultaneous addition of enzymes in reactions containing PET as substrate (8mg). PET powder was obtained by grinding the PET bottle in a ball mill (Tecnal). For non-simultaneous assays, the substrate was pretreated with *Af*LPMO9A (0.5, 2 or 4 µM) observing the same conditions highlighted in "Enzymatic Assays - LPMO". The supernatant was boiled and the residual PET was washed with Milli-Q water and sonicated three times (10 min each). Then, *Is*PETase (0.05 µM) was added to the supernatant or residual PET and the reactions were carried out in 0.05 M sodium phosphate buffer, pH 7.2, at 30oC / 400 rpm / 120 h. In simultaneous assays, *Af*LPMO9A and *Is*PETase were added simultaneously in reactions containing powdered PET. The A/LPMO9A concentration varied (0.05-4 µM) while the *Is*PETase concentration was kept fixed (0.05µM) in 0.05 M sodium phosphate buffer, pH 6.0, 37 °C / 850 rpm / 120 h.

The supernatants were boiled at 99 °C for 5 min, dried in SpeedVac concentrators (Eppendorf) and resuspended in a mixture of 20% methanol: 80% DMSO. The concentration of mono (2-hydroxyethyl) terephthalate (MHET) and terephthalic acid (TPA) released by *Is*PETase was determined by HPLC. Reactions only with *Is*PETase were used as controls for non-simultaneous and simultaneous assays. All reactions were performed in a volume of 600 uL, in triplicate. The *Is*PETase concentration adopted in all synergy assays was set at 0.05 µM.

In non-simultaneous synergy assays, PET powder was pretreated with *Af*LPMO9A + AscAc (pH 6.0 / 37°C / 48 h), washed/sonicated three times and added with *Is*PETase (pH 7.2 / 30° C / 120 h). Pretreatment with 0.5, 2 and 4 µM *Af*LPMO9A + AscAc improved MHET release by 20, 16 and 23%, respectively, and TPA by 34, 64 and 49%, respectively, by *Is*PETse.

Synergy between *Af*LPMO9A and *Is*PETase was also found when the substrate was first treated with 0.5, 2 or 4 µM of *Af*LPMO9A in the absence of AscAc, in agreement with atomic force microscopy data regarding LPMO modification of PET, even without the addition of the electron donor. However, the improvement in MHET and TPA release was lower than that achieved in the AscAc reactions: 9-24% and 18-40%, respectively, revealing the importance of free electron donors in the reaction to promote *Af*LPMO9A activity.

In addition to the substrate, supernatants from reactions with *Af*LPMO9A were boiled and added with *Is*PETase, but neither MHET nor TPA were detected in this condition.

Given the similar activity of IsPETase at pH 7.2 / 30 °C (1.709 + 0.06 U / mg protein) and pH 6.0 / 37 °C (1.969 + 0.02 U / mg protein) using α - naphthyl acetate (N-ace) as substrate, the posterior condition was adopted in the synergy tests with the simultaneous addition of *Is*PETase and *Af*LPMO9A. Reactions with *Is*PETase and *Af*LPMO9A in a 1:1 ratio (without AscAc) resulted in a slight improvement of MHET (10%) and TPA (8%). The successive addition of *A*fLPMO9A at 1:10, 1:15, 1:20 and 1:35 decreased IsPETase activity.

The addition of AscAc to *Is*PETase affected its activity on PET and naphthyl esters, which can be attributed to a drop in reaction pH from 6.0 to 4.9. However, a 20% improvement in MHET and TPA concentration was recorded after addition of A/LPMO9A 1:1 in this condition.

### Example 8: Testing AfLPMO9A activity on other substrates

Further to the PET, high (HDPE) and low density (LDPE) polyethylenes were evaluated as substrates for *Af*LPMO9A activity.

The surface modification caused by AfLPMO9A on juice bottles and plastic bags, both made of HDPE, is evident in comparison to controls with AscAc. Erosion was more subtle than that caused in a PET bottle and better verified in topographic images of atomic force microscopy 1x1 µm, which was confirmed by the mean diameter (34 and 17 nm) and depth (4.8 and 4.4 nm ) of the eroded areas in the juice bottle and plastic bag, respectively. No LDPE activity was detected under the tested conditions.

## Claims

1. Enzymatic composition for use in the degradation of polyester, **characterized by** comprising
at least one lytic polysaccharide monooxygenase selected from an enzyme comprising the amino acid sequence having at least 60% identity with SEQ ID NO: 2, an enzyme comprising the amino acid sequence having at least 60% identity with SEQ ID NO: 5, or a mixture thereof; and
an acceptable vehicle.

2. Enzymatic composition, according to claim 1, **characterized in that** the enzyme comprising the amino acid sequence that has at least 60% identity with SEQ ID NO: 2 is present in a concentration between approximately 0.001% and approximately 0.100% by total weight of the composition, preferably between approximately 0.002% and approximately 0.005% by total weight of the composition.

3. Enzymatic composition, according to claim 2, **characterized in that** the enzyme comprising the amino acid sequence that has at least 60% identity with SEQ ID NO: 2 is *Kp*LPMOA10 (AA10) isolated from *Kitasatospora papulosa.*

4. Enzymatic composition, according to claim 1, **characterized in that** the enzyme comprising the amino acid sequence SEQ ID NO: 5 is present in a concentration between approximately 0.001% and approximately 0.100% in total weight of the composition, preferably between approximately 0.002% and approximately 0.005% by total weight of the composition.

5. Enzymatic composition, according to claim 4, **characterized in that** the enzyme comprising an amino acid sequence that has at least 60% identity with SEQ ID NO: 5 is the *Af*LPMOA9 (AA9) isolated from *Aspergillus fischeri.*

6. Enzymatic composition, according to any one of claims 1 to 5, **characterized in that** it additionally comprises at least one cutinase.

7. Enzymatic composition, according to claim 6, **characterized in that** the cutinase is a PETase.

8. Enzymatic composition, according to claim 7, **characterized in that** the PETase is in a concentration between approximately 0.0005% and approximately 0.0500% in total weight of the composition, preferably between approximately 0.0010% and approximately 0 .0025% by total weight of the composition.

9. Enzymatic composition, **characterized by** comprising:
approximately 0.003% of *Kp*LPMOA10;
approximately 0.003% of *Af*LPMOA9; and
an acceptable vehicle.

10. Enzymatic composition, according to claim 9, **characterized by** further comprising 0.002% of a PETase.

11. Use of the enzymatic composition as defined in any one of claims 1 to 10, **characterized in that** it is used in the degradation of polyethylene terephthalate (PET).

12. Use, according to claim 11, **characterized in that** the PET is selected from liquid bottles, food packaging, window films, X-ray films, fabric fibers, a mixture of these and any other materials mostly made of PET, without limitations.

13. Method of PET degradation, **characterized by** using at least one lytic polysaccharide monooxygenase selected from an enzyme comprising an amino acid sequence having at least 60% identity with SEQ ID NO: 2, an enzyme comprising a amino acid sequence that shows at least 60% identity with SEQ ID NO: 5, or a mixture thereof.

14. Method, according to claim 13, **characterized in that** it is carried out in an aqueous medium, wherein the aqueous medium comprises a buffer.

15. Method according to claim 13, **characterized in that** it comprises the addition of at least one electron-donating substance, wherein the electron-donating substance is ascorbic acid.

16. Method according to claim 13, **characterized in that** the enzyme comprising an amino acid sequence that has at least 60% identity with SEQ ID NO: 2 is *Kp*LPMOA10 (AA10) isolated from *Kitasatospora papulosa.*

17. Method, according to claim 16, **characterized in that** KpLPMOA10 (AA10) is used in an amount ranging from approximately 0.01 to approximately 50.00 milligrams of enzyme per gram of PET, preferably in an amount ranging from approximately 2.0 to approximately 20.0 milligrams of enzyme per gram of PET.

18. Method, according to claim 13, **characterized in that** the enzyme comprising an amino acid sequence that has at least 60% identity with SEQ ID NO: 5 is *Af*LPMOA9 (AA9) isolated from *Aspergillus fischeri.*

19. Method, according to claim 18, **characterized in that** *Af*LPMOA10 (AA10) is used in an amount ranging from approximately 0.01 to approximately 50.00 milligrams of enzyme per gram of PET, preferably in an amount ranging from approximately 2.0 to approximately 20.0 milligrams of enzyme per gram of PET.

20. Method according to any one of claims 13 to 19, **characterized in that** it additionally uses at least one cutinase, wherein the cutinase is a PETase.

21. Method, according to claim 20, **characterized in that** PETase is used in an amount ranging from approximately 0.005 to approximately 25.00 milligrams of PETase per gram of PET, preferably approximately 1.0 to approximately 10.0 milligrams of PETase per gram of PET.

22. Method according to any one of claims 13 to 21, **characterized in that** it is conducted at pH values ranging from approximately 4.0 to approximately 9.0, preferably ranging from approximately 5.5 to approximately 7.5.

23. Method according to any one of claims 13 to 21, **characterized in that** it is conducted at a temperature ranging from approximately 20 °C to approximately 60 °C, preferably 30 °C to approximately 40 °C.

24. Method according to any one of claims 13 to 21, **characterized in that** it is carried out in a period ranging from approximately 5 minutes to approximately 48 hours, preferably in a period ranging from approximately 60 minutes to approximately 48 hours.

25. Use of at least one lytic polysaccharide monooxygenase **characterized by** being in the enzymatic degradation of polyester, in which said lytic polysaccharide monooxygenase degrades said polyester without the aid of one or more canonical enzymes, in which the canonical enzymes are a PETase or a mixture of PETases.

26. Use, according to claim 25, **characterized in that** the lytic polysaccharide monooxygenase comprises an amino acid sequence that has at least 60% identity with SEQ ID NO: 22.

27. Use, according to claim 26, **characterized in that** the lytic polysaccharide monooxygenase comprises an amino acid sequence that has at least 60% identity with SEQ ID NO: 2 obtained from *Kitasatospora papulosa.*

28. Use, according to claim 25, **characterized in that** the lytic polysaccharide monooxygenase comprises an amino acid sequence that has at least 60% identity with SEQ ID NO: 5.

29. Use according to claim 28, **characterized in that** the lytic polysaccharide monooxygenase comprising an amino acid sequence that has at least 60% identity with SEQ ID NO: 5 obtained from *Aspergillus fischeri.*

30. Use, according to any one of claims 25 to 29, **characterized in that** the polyester comprises polyethylene terephthalate (PET).

31. Use, according to claim 30, **characterized in that** PET is selected from liquid bottles, food packaging, window films, X-ray films, fabric fibers, a mixture of these and any other materials mostly made of PET, without limitations.
